# FASCICULE DE BREVET EUROPEEN

(11) **EP 4 222 480 B1**
(45) Date de publication et mention de la délivrance du brevet: **10.06.2026**
(21) Numéro de dépôt: 21794605.2
(22) Date de dépôt: 28.09.2021
(51) Int. Cl.: G01N 21/3563, G01N 21/33, G01N 21/64, G01N 21/47, G01N 21/94, G01J 3/28, E21B 49/00, G01N 33/24, G01V 8/02

(54) **PROCEDE D'ANALYSE DE LA POLLUTION DES SOLS**
VERFAHREN ZUR ANALYSE VON BODENVERSCHMUTZUNG
METHOD FOR ANALYZING SOIL POLLUTION

(30) Priorité: 30.09.2020 FR 2009996
(43) Date de publication de la demande: 09.08.2023
(73) Titulaire: Tellux, 76480 Saint-Paër (FR)
(72) Inventeur: VAN EXEM, Antonin, 76480 SAINT-PAËR (FR); HONEINE, Paul, 76100 ROUEN (FR); MIGNOT, Mélanie, 76380 CANTELEU (FR)
(74) Mandataire: IP Trust
(86) Numéro de dépôt international: PCT/FR2021/051667
(87) Numéro de publication internationale: WO 2022/069827

(56) Documents cités:
- NAWAR SAID ET AL: "Estimating the soil clay content and organic matter by means of different calibration methods of vis-NIR diffuse reflectance spectroscopy", SOIL AND TILLAGE RESEARCH, vol. 155, 1 January 2016 (2016-01-01), NL, pages 510 - 522, XP093218561, ISSN: 0167-1987, DOI: 10.1016/j.still.2015.07.021
- ZOU BIN ET AL: "Multisource spectral-integrated estimation of cadmium concentrations in soil using a direct standardization and Spiking algorithm", SCIENCE OF THE TOTAL ENVIRONMENT, ELSEVIER, AMSTERDAM, NL, vol. 701, 1 November 2019 (2019-11-01), XP085927265, ISSN: 0048-9697, [retrieved on 20191101], DOI: 10.1016/J.SCITOTENV.2019.134890
- SCAFUTTO REBECCA DEL'PAPA MOREIRA ET AL: "Quantitative characterization of crude oils and fuels in mineral substrates using reflectance spectroscopy: Implications for remote sensing", INTERNATIONAL JOURNAL OF APPLIED EARTH OBSERVATION ANDGEOINFORMATION, ELSEVIER, AMSTERDAM, NL, vol. 50, 12 April 2016 (2016-04-12), pages 221 - 242, XP029516478, ISSN: 0303-2434, DOI: 10.1016/J.JAG.2016.03.017
- KOPEL DANIELLA ET AL: "Spectroscopy as a Diagnostic Tool for Urban Soil", WATER, AIR, & SOIL POLLUTION, KLUWER ACADEMIC PUBLISHERS, DORDRECHT, NL, vol. 226, no. 8, 2 July 2015 (2015-07-02), pages 1 - 18, XP035506143, ISSN: 0049-6979, [retrieved on 20150702], DOI: 10.1007/S11270-015-2442-2

## Description

La présente invention concerne le domaine de l'analyse de contamination de sols par des polluants organiques.

Les sources de contamination de sols sont variées : l'utilisation d'engrais, de pesticides, de rejets d'un site industriel, la proximité d'un incinérateur, d'un lieu de stockage de déchets, de rejets de résidus de médicaments par les déjections d'animaux d'élevage, d'hydrocarbures...La pollution des sols, terrains, jardins ou aires de jeux peut provenir d'agents chimiques : dioxines, PCB ou de métaux toxiques extrêmement dangereux pour la santé.

L'origine de ces pollutions peut être accidentelle (déversement ou dépôts ponctuels de polluants par négligences, disfonctionnement d'une installation industrielle, accident d'une installation ou d'un engin de transport de matières polluantes), avec une grande quantité de polluant déversée, ou chronique (apport continu de contaminants par fuite ou lessivage, dont les effets cumulés peuvent être plus importants et plus insidieux que ceux d'une pollution accidentelle).

Un sol pollué peut être la cause d'intoxications lors de la consommation de fruits, de légumes du jardin. La bioaccumulation des polluants contenus dans les sols par les végétaux et les animaux font d'eux, les polluants les plus dangereux pour la santé humaine et animale. Ils ont en effet la particularité de contaminer la chaine alimentaire (dioxines, PCB, radioactivité...). Les polluants contenus dans les sols peuvent également provoquer des irritations de la peau et du système respiratoire. Ils sont aussi responsables de troubles cardiaques et neurologiques, de la perte de fertilité, de troubles de développement du fœtus, et à l'origine de certains cancers.

Pour évaluer la présence d'une contamination et qualifier et quantifier la nature des polluants, il est usuel de procéder à des prélèvements, par exemple par carottage et de soumettre ces prélèvements à un laboratoire d'analyse physicochimique. Le « Guide méthodologique pour l'analyse des sols pollués » édité en février 2000 sous la référence BRGM/RP-50128-FR présente de manière détaillée les techniques pour l'analyse de sols pollués.

Ces analyses nécessitant des ressources et des compétences scientifiques de haut niveau, on a aussi proposé d'automatiser tout ou partie de ces analyses.

Pour un site donné, les étapes d'analyses classiques des sols impliquent de :
1. Faire venir une entreprise de sondage pour réaliser les carottes ;
2. Envoyer des échantillons à des laboratoires d'analyses ;
3. Attendre le retour des analyses ;
4. Interpréter ces analyses sous forme de cartes ;
5. Rédiger un rapport de recommandation.

La durée minimum d'une telle analyse est de 6 à 8 semaines. À la suite de ces analyses, le rapport risque souvent de montrer des incertitudes significatives à cause de l'hétérogénéité dans le sol étudié et ainsi recommander une nouvelle campagne d'échantillonnage. En effet, l'échantillonnage initial est souvent insuffisant car les distances entre les points d'échantillonnage sont trop importantes (politique de coût). Il faut alors repartir pour la boucle décrite dans la problématique précédente pour une ou plusieurs fois. Le retard dû à cette problématique est d'au moins un mois.

Enfin, lors de la dépollution, le terrain est creusé par l'entreprise de dépollution et des pollutions non prévues peuvent être détectées. Il s'en suit un arrêt des travaux et on repart avec un ré-échantillonnage et analyse comme décrit ci-dessus. Pendant ce temps, les déblais sont stockés temporairement sur le site avant de connaître la nature de la pollution et de les envoyer vers les centres de retraitement compétents. Dans ce cas, un retard supplémentaire de 15 jours au moins est observé et des surcoûts importants liés à l'immobilisation des ouvriers et des machines, ainsi que le retraitement des terres polluées non diagnostiquées initialement.

### État de la technique

On connaît dans l'état de la technique l'article de Bin Zou, Xiaolu Jiang, Huihui Feng, Yulong Tu, Chao Tao, « Multisource spectral-integrated estimation of cadmium concentrations in soil using a direct standardization and Spiking algorithm » paru dans Science of The Total Environment, Volume 701, 2020, 134890, ISSN 0048-9697, concernant le domaine de la la télédétection de bas niveau et par satellite à grande échelle et plus précisément l'étude de la réponse spectrale exacte du cadmium (Cd) dans le sol et présente une nouvelle méthode en combinant les algorithmes de normalisation directe (DS) et de Spiking pour intégrer des spectres multisources afin d'améliorer la précision de l'estimation de la concentration de Cd.
Cet article concerne l'analyse de la présence d'un métal lourd, le Cadmium, dans des échantillons dans lequel il est présent. Cet article n'apporte aucun enseignement sur la caractérisation de polluants inconnus, notamment organiques, sur un échantillon prélevé par carottage sur le terrain.
On connaît aussi la publication « Scafutto, Rebecca & Souza Filho, Carlos. (2016). Quantitative characterization of crude oils and fuels in mineral substrates using reflectance spectroscopy: Implications for remote sensing. International Journal of Applied Earth Observation and Geoinformation. 50. 221-242. 10.1016/j.jag.2016.03.017. » concernant la surveillance environnementale des fuites de pétrole et de carburants à l'aide de la télédétection multispectrale, hyperspectrale et ultra-spectrale proximale et lointaine. Cette publication est basée sur la mesure des propriétés de réflectance spectrale infrarouge proche et courte de plusieurs substrats minéraux imprégnés de pétrole brut, de diesel, d'essence et d'éthanol à l'aide de l'analyse en composantes principales (ACP) et de la régression des moindres carrés partiels (PLS). Ces caractéristiques ont été utilisées pour la détermination qualitative et quantitative du contaminant imprégné dans les substrats. Des longueurs d'onde spécifiques, où se produisent des bandes d'absorption clés, ont été utilisées pour la caractérisation individuelle des huiles et des carburants. L'intensité de ces caractéristiques peut être corrélée à l'abondance du contaminant dans les mélanges. La taille des grains et la composition du substrat imprégné influencent directement la variation des signatures spectrales.
On connaît aussi l'article « Kopel, Daniella & Brook, Anna & Wittenberg, Lea & Malkinson, Dan. (2015). Spectroscopy as a Diagnostic Tool for Urban Soil. Water, Air, and Soil Pollution. 226. 10.1007/s11270-015-2442-2. » concernant la détection de l'activité spectrale (SA) dans une approche hiérarchique structurée pour identifier les caractéristiques spectrales dominantes. La méthode développée est adoptée par plusieurs outils en production, notamment la normalisation de suppression de continuum, guidée par la généralisation polynomiale, et les algorithmes de vraisemblance spectrale : projection de sous-espace orthogonal (OSP) et analyse itérative de mélange spectral (ISMA).

On connaît également l'article " Estimating the soil clay content and organic matter by means of different calibration methods of vis-NIR diffuse reflectance spectroscopy"

Said Nawara.b, et Al. Soil & Tillage Research 155 (2016) 510-522, concernant l'estimation de la teneur en argile et en matière organique des sols à l'aide de la spectroscopie de réflectance dans le visible et le proche infrarouge (VNIRRS). L'objectif principal est d'optimiser les méthodes de calibration pour améliorer la précision des prédictions des propriétés du sol. Trois techniques de régression multivariée sont comparées : la régression par moindres carrés partiels (PLSR), la régression par vecteurs de support (SVR) et les splines de régression adaptative multivariée (MARS).

L'étude est réalisée sur 102 échantillons de sols collectés dans la plaine d'El-Tina en Égypte, scannés avec un spectroradiomètre portable couvrant un spectre de 350 à 2500 nm. Différentes méthodes de prétraitement des spectres sont évaluées, notamment le lissage de Savitzky-Golay, la dérivation spectrale et la normalisation.

### Inconvénients de l'art antérieur

Les solutions de l'art antérieur ne permettent pas d'atteindre un niveau de précision et de fiabilité suffisant lorsque l'analyse est réalisée directement sur le site par une méthode spectrométrique et notamment par imagerie hyperspectrale.

Par ailleurs, pour répondre aux besoins de terrain, il est important de pouvoir fournir une information qualifiée sur la teneur en polluants en fonction de la profondeur, afin de pouvoir optimiser le traitement du terrain, et les solutions basées sur l'analyse d'images prises à distance sont mal adaptées.

### Solution apportée par l'invention

Afin de remédier à ces inconvénients, l'invention concerne selon son acception la plus générale à un procédé d'analyse de la contamination de sols par des polluants, notamment organiques, comme défini dans la revendication 1. Le procédé selon l'invention comporte :
- une séquence d'apprentissage consistant à procéder à l'analyse d'une pluralité d'échantillons de référence, et d'enregistrement dans une base de données d'apprentissage
   ∘ a) de la signature spectrale de réflexion acquises par l'analyse spectrale
   ∘ b) des valeurs connues des variables représentatives des contaminants présents dans chacun desdits échantillons de référence
   ∘ c) des valeurs connues des variables représentatives des substrats de chacun desdits échantillons de référence
- une séquence de calibration d'un équipement d'analyse sur le terrain par rapport audit premier équipement, ledit équipement de terrain comportant une source lumineuse et un capteur spectral,
- des séquences d'analyse d'un échantillon du sol d'un site géologique consistant à acquérir la signature de réflexion et de la photoluminesce dudit échantillon à l'aide dudit équipement de terrain ainsi calibré,
- et à procéder à l'estimation de la caractérisation des polluants par traitement de ladite signature par un moteur d'apprentissage exploitant les données de la base de données constituée lors de la séquence d'apprentissage.

Avantageusement le système d'analyse selon l'invention comporte une sonde comportant au moins une fibre optique pour transmettre la lumière entre la zone d'analyse de ladite sonde d'une part, et la source lumineuse, notamment au Xénon ou Halogène, et ledit au moins un capteur d'autre part.

Selon une variante optionnelle, ledit équipement comporte en outre un moyen d'analyse physico-chimique.

### Description d'un exemple non limitatif de l'invention

La présente invention sera mieux comprise à la lecture de la description qui suit, se référant aux dessins annexés concernant des exemples non limitatifs de réalisation, où :

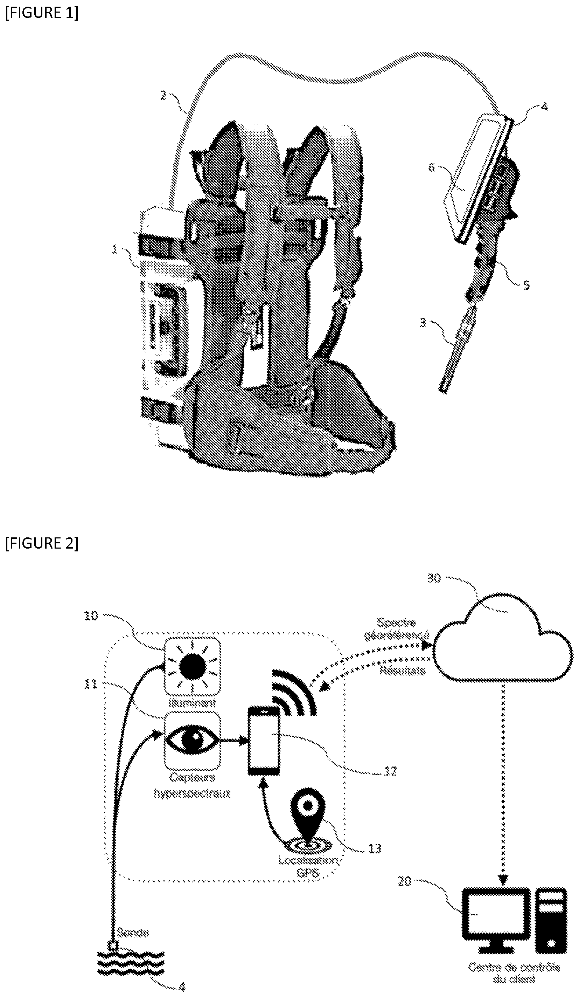

[FIGURE 1] la figure 1 représente l'architecture matériel d'un exemple de mise en œuvre de l'invention
[FIGURE 2] la figure 2 représente le schéma fonctionnel de l'invention
[FIGURE 3] la figure 3 représente l'architecture fonctionnelle
[FIGURE 4] la figure 4 représente le schéma optique d'une variante de réalisation du système optique.

### Contexte général de l'invention

L'invention concerne la caractérisation de prélèvements de sols par carottage pour déterminer la présence qualitative et quantitative de constituants d'intérêt (polluants, notamment plastiques, hydrocarbures, métaux...) pour fournir un outil de mesure en temps réel de la nature des polluants sur les chantiers de valorisation de terres polluées.

Le but est d'apporter une solution pour fournir si possible en temps réel et sur site une carte détaillée de la pollution afin de faciliter et sécuriser la mise en œuvre d'un tri sélectif des déblais en fonction de leur niveau de pollution, directement sur chantier grâce à un outil de mesure en temps réel. Le résultat est une cartographie en trois dimensions du terrain pour fournir une information géolocalisée sur la pollution, en coordonnées x,y avec une résolution de quelques mètres carrés à quelques centaines de mètres carrés, et en profondeur z, et permettre ensuite de décider en toute sécurité des mesures de dépollutions optimales. Optionnellement, l'analyse de prélèvements par carottage peut être complétée par une analyse additionnelle à distance, par une caméra hyper spectrale afin de quantifier les polluants livrés dans des centres d'analyse pour contrôler, en temps réel, les terres polluées qu'ils réceptionnent. Ainsi, le système proposé garantit la qualité de la terre, identifie ses polluants, afin de permettre son recyclage.

A cet effet, l'invention permet l'instrumentation des machines de forages et engins de chantier pour permettre d'intégrer d'analyses en temps réel et accélérer le processus en supprimant les étapes itératives et les sources d'incertitudes financières.

Ainsi, lorsqu'une pollution non identifiée initialement est détectée, des mesures en temps réel permettraient d'orienter le déblai vers la filière de valorisation correspondante sans le délai d'analyse en laboratoire (optimisation des prises de décision en temps réel).

### Architecture matérielle

L'équipement selon un exemple de mise en œuvre de l'invention est constitué par un équipement de terrain (1), portable dans l'exemple décrit comportant un boîtier de type sac-à-dos comportant une source à large spectre lumineux, par exemple une lampe au Xénon (10), ainsi qu'une alimentation, par exemple des batteries. La source lumineuse (10) est associée par une fibre optique (2) à une lance (3) qui transmet la lumière vers le sol et réfléchit la lumière vers des capteurs (11) dont les signaux électriques sont transmis à un calculateur qui peut être logé dans le boitier ou fixé sous la forme d'une tablette (4) sur la poignée (5) de la lance (3).

Les capteurs sont des capteurs hyperspectraux présentant une plage de sensibilité comprise entre 100 microns et 200 nanomètres. Il peut s'agit d'une caméra hyperspectrale, ou d'une caméra multispectrale, ou d'un assemblage de capteurs formant un capteur multispectrale composite.

La lance comporte une ou plusieurs fibres optiques pour transmettre la lumière émise par la source (10) à une extrémité de mesure, et pour recueillir la lumière réfléchie ou la lumière de fluorescence de l'échantillon vers l'extrémité de la lance (3). La fibre peut comporter à son extrémité une lentille ou une optique de collimation.

La tablette (4) comporte une interface tactile (6) munie d'un module (13) de géolocalisation (GPS ou Galileo) et d'une carte de téléphonie mobile SIM 5G.

Cet équipement comporte en outre un calculateur (12) et des moyens de radiocommunication qui permettent d'une part, de traiter les données et fournir en temps réel un diagnostic de l'état du sol et de la pollution (caractéristiques lithologiques, type de polluants, quantité de polluants, cartographie 3D du sol...) ; et d'autre part, de communiquer les données vers un espace de stockage Cloud (30). Les données sont disponibles en local pour la prise de décision en temps réel mais également sur une plateforme en ligne pour permettre au chef de projet équipé d'un terminal connecté (20), présent au centre de contrôle, de suivre les opérations en direct et de collecter les données acquises sur le terrain.

### Mise en œuvre de l'invention

Un carottage est réalisé par un opérateur équipé de l'équipement susvisé, sur un site suspecté de contamination, l'emplacement du forage est sondé par carottage ou taraudage par un instrument muni d'un module de géolocalisation, et ses coordonnées géographiques sont enregistrées. Plusieurs sections d'environ 1 mètre de profondeur sont prélevées au droit du forage et plusieurs forages sont réalisés sur un site.

L'analyse spectrale de l'ensemble des carottes est réalisée. Pour cela on retire les demi-carottes ouvertes en deux dans la longueur, et on procède à l'acquisition de données sur plusieurs demi-carottages simultanée ce qui est possible du fait de la vitesse d'acquisition rapide de l'imagerie. L'extraction de sous-images correspondantes à une demi-carotte est facilitée par le positionnement de QR-code reconnues numériquement et disposés sur les angles des sections. L'imagerie peut être réalisée sur-site avec une caméra dans un véhicule ou un container. Un modèle prédictif pré-entrainé peut être utilisé à ce stade, à partir d'une quantité de données suffisantes dans la base de données pour que le modèle pré-entrainé (sur ces données) suffise à produire un diagnostic en temps réel.

La sélection de sous-échantillons est réalisée à partir des données d'imagerie. Des méthodes statistiques, supervisées (Machine Learning) ou non-supervisées (end-members extraction, features extraction, novelty detection, etc.), sont utilisées pour sélectionner les zones où prélever ces sous-échantillons afin de représenter l'hétérogénéité des formations géologiques présentes sur le site. Des données de spectromètre peuvent être utilisées en alternative, le prélèvement des sous-échantillons peut être réalisé immédiatement (cas des polluants volatils) ou réalisé plus tard en laboratoire (les demi-carottes sont refermées hermétiquement avec du film plastique et stockées en chambre froide pour conservation).

Les analyses chimiques consistent à réaliser l'extraction des polluants par différentes méthodes, par solvant (eau, hexane, éther), par agitations, par micro extraction en phase solide, par microonde, par headspace, etc. Les techniques d'analyses réalisées sont la chromatographie (ICP-AES, ionique, HS-GS/MS). Les analyses sont réalisées sur-site (dans un container) ou en laboratoire, sur des sous-échantillons de carottages ou des échantillons fournis et/ou non sélectionnables (ex, prélevés par tarière).

L'entraînement de modèle prédictif est réalisé à partir d'une base de données et d'échantillons de référence. Un premier modèle de traitement consiste à convertir des données brutes issues du capteur en réflexion. Cette étape se nomme normalisation en référence à la méthode actuelle qui utilise les données brutes mesurées sur un matériel de référence de réflexion >99% (Spectralon(R)) et des données de bruit électronique mesurées sans illuminant (source) pour normer les données des échantillons entre ces deux spectres (i.e. 0 et 100% de réflexion). Afin de supprimer ces mesures en amont de l'acquisitions, un modèle est généré à partir de l'enregistrement préalable de ces données brutes de référence. Des données brutes mesurées sur 8 matériels de référence (de 2% à 99% de réflexion) et le bruit électronique sont utilisés, un modèle peut être entrainé pour chaque combinaison de paramètres d'un appareil. Un deuxième niveau de traitement prédit les variables d'intérêt (composition du sol, présence de polluants et quantité de polluant) à partir de la réflexion d'un échantillon. Plusieurs bases de données d'entrainement sont utilisées : des bases d'entrainement publiées (librairie spectrale de composés pure, ex : USGS Spectral Library), des données produites sur des échantillons artificiels produits en laboratoire ou des données produites sur des échantillons analysés en laboratoire. Dans le cas où un lot d'échantillons analysés serait issu d'un site en particulier, un modèle peut être entraîné sur ce lot uniquement ou ce lot peut être utilisé pour améliorer un modèle pré-entraîné sur base existante par méthode d'apprentissage par transfert.

Le traitement de données s'applique aux données d'imagerie, les données issues de sous-échantillons analysés permettent d'interpréter ou d'affiner une première interprétation des carottages. Le modèle généré avec les données du spectromètre permet de produire des analyses en temps réel, y compris des analyses référencées par rapport à des données de laboratoires certifiés COFRAC. Le couplage imagerie sur-site puis spectrométrie répond à la phase de diagnostic d'un site, puis à la phase de travaux. Un tri sélectif des terres excavées en fonction de leur classe de déchet est possible. Un contrôle qualité des terres recyclées en centre de stockage est réalisé à partir d'échantillons fournis, artificiel ou semi-artificiel (échantillon fourni dilué ou dopé artificiellement pour couvrir une gamme de concentrations plus importante et améliorer le modèle).

La cartographie des résultats constitue un troisième niveau d'interprétation à partir d'un modèle de Machine Learning. Une interpolation dans l'espace des variables d'intérêt mesurées sur différents forages permet de générer une carte. Les méthodes de modélisation des processus Gaussiens sont utilisées. Les données géophysiques mesurées au moment des forages sont utilisées dans la cartographie par méthodes de fusion de données.

Les mesures in-situ peuvent être envisagées. L'acquisition de données par spectromètre fibré permet de sonder le sol et de produire un profil des variables d'intérêt en fonction de la profondeur. (Remarque : on a fait une différence entre in-situ et sur-site : L'imagerie peut être réalisée sur-site avec une caméra dans un véhicule ou un container. Le terme « in-situ » concerne l'exploitation d'un équipement comprenant une sonde fibrée avec un ou plusieurs spectromètres en version portable précédemment décrite).

### Schéma fonctionnel

La première étape (100) consiste à procéder à un prélèvement d'une carotte d'une longueur déterminée par la profondeur de terrain à analyser à l'aide de la sonde (4). Le carottage est géolocalisé par le module GPS (13) de l'équipement.

L'étape suivante (200) consiste à procéder à une analyse de la carotte sur la longueur de la carotte par mesure de la réflexion hyperspectrale mesurée par les capteurs (11) lors de l'éclairage avec la source (10).

Les données obtenues lors de l'étape d'analyse sont enregistrées localement et dans le cloud, et font l'objet ensuite d'une étape (300) de sélection d'un sous-ensemble d'échantillons pour réaliser soit un entraînement du modèle (étapes 400, 500, 600), soit une analyse sur site (étapes 450, 550, 650).

### Variante de l'étape d'apprentissage

L'apprentissage peut être mutualisé à partir d'analyses en laboratoire, avec un équipement, munie d'une caméra hyperspectrale performante, pour enregistrer les signatures spectrales d'un nombre important d'échantillons de référence, et fournir une base de données accessible à une pluralité d'équipements de terrain munis de capteurs moins performants et moins onéreux.

Afin de prendre en compte les différences techniques et optiques, chaque équipement de terrain est calibré à l'aide d'échantillons de référence dont la signature spectrale a préalablement été enregistrée dans la base de données. On calcule une fonction de correction permettant d'exploiter le contenu de la base de données avec un équipement différent de celui ayant servi à l'analyse initiale.

Les échantillons se distinguent d'une part par la nature du substrat, et d'autre part par la nature des polluants présents.

Les substrats sont caractérisés par des méta-descripteurs en fonction de variables telles que :
- La nature chimique des constituants minéraux et organiques
- La teneur en eau
- La teneur en oxyde
- Le pH
- La granulométrie
- L'appartenance à une ou plusieurs classes minérales selon la classification de Strunz
- Le potentiel d'oxydo-réduction REDOX.

Le substrat de référence peut être caractérisé par des analyses physico-chimiques. Il peut aussi être préparé à partir de composants prédéterminés pour préparer des substrats par assemblage.

Les polluants de référence sont caractérisés par leur composition chimique.

On procède ensuite, pour chacun des échantillons de référence à l'enregistrement de la signature spectrale en le soumettant à un éclairage par une source lumineuse, par exemple une lampe au Xénon, et on capte la lumière réfléchie ainsi que la lumière émise par photoluminescence dans une plage de longueur d'onde allant de l'infrarouge thermique jusqu'à l'ultraviolet UVC. Les données sont enregistrées pour chacun des échantillons avec un identifiant de l'échantillon de référence et les caractéristiques physico-chimiques.

Selon une alternative préférée on procède d'abord à l'acquisition spectral de l'échantillon ou toute la carotte, puis on extrait un (sous-)échantillon (ou plusieurs) pour l'analyse physico-chimique.

### Acquisition spectrale

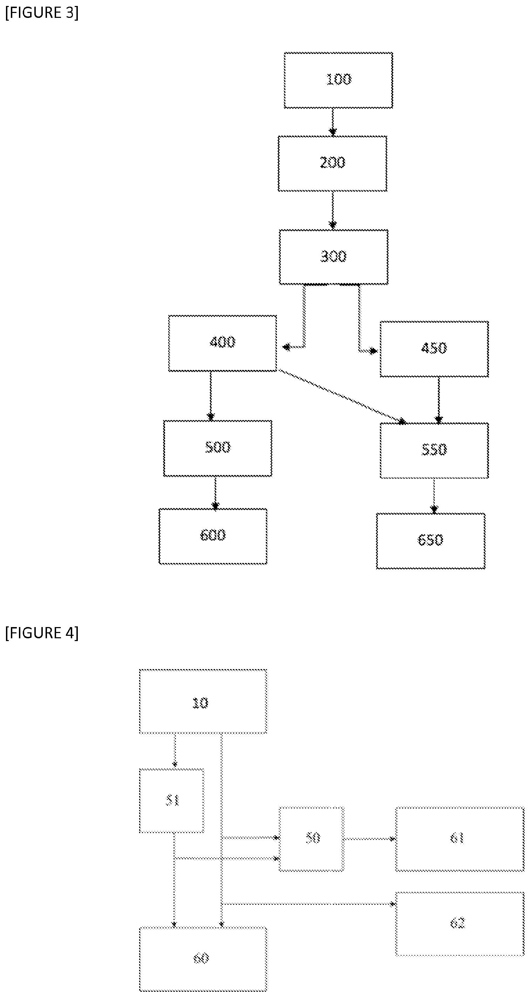

La figure 4 représente le schéma optique d'une variante de réalisation du système optique. La configuration consiste en 2 canaux séparés exploités par un faisceau de fibres connecté à une lampe Xénon (10) qui irradie des échantillons de sol (60) et recueille la lumière réfléchie dans chaque canal au moyen d'un interrupteur optique (50). Un monochromateur (51) placé sur le trajet optique fournit un faisceau secondaire d'excitation de la fluorescence.

Le premier canal de réflectance est destiné à la collecte des photons simultanément sur deux spectromètres distincts :
- Un spectromètre (61) dans la bande visible et ultraviolet
- Un spectromètre (62) dans la bande infrarouge.
Le deuxième canal est destiné à la collecte des photons de fluorescence dans la gamme UV-Visible-PIR : la lumière incidente monochromatique est sélectionnée au moyen d'un monochromateur connecté à la lampe Xénon et les photons sont collectés sur le spectromètre UV-Visible-PIR (61).

## Revendications

1. Procédé d'analyse de la contamination de sols par des polluants, notamment organiques, pour déterminer la présence qualitative et quantitative de constituants d'intérêt constituant des polluants et comprenant notamment des plastiques, des hydrocarbures et des métaux par analyse hyperspectrale de la réflexion et de la photoluminescence **caractérisé en ce que** ladite analyse est réalisée avec un premier équipement par l'éclairage d'un échantillon par une source lumineuse et par au moins un capteur spectral sensible sur un spectre allant de l'infrarouge thermique à l'ultraviolet, et **en ce qu'**il comporte :
- une séquence d'apprentissage consistant à procéder à l'analyse d'une pluralité d'échantillons de référence, et d'enregistrement dans une base de données d'apprentissage
∘ a) de la signature spectrale de réflexion acquises par l'analyse spectrale
∘ b) des valeurs connues des variables représentatives des contaminants présents dans chacun desdits échantillons de référence
∘ c) des valeurs connues des variables représentatives des substrats de chacun desdits échantillons de référence
- une séquence de calibration d'un équipement d'analyse sur le terrain par rapport audit premier équipement, ledit équipement de terrain comportant une source lumineuse et un capteur spectral,
- des séquences d'analyse d'un échantillon du sol d'un site géologique consistant à acquérir la signature de réflexion et de la photoluminescence dudit échantillon à l'aide dudit équipement de terrain ainsi calibré,
- et à procéder à l'estimation de la caractérisation des polluants par traitement de ladite signature par un moteur d'apprentissage exploitant les données de la base de données constituée lors de la séquence d'apprentissage.

2. Procédé d'analyse de la contamination de sols par des polluants, notamment organiques selon la revendication 1 **caractérisé en ce que** l'on procède, lors de l'analyse d'un site, à au moins un carottage et **en ce qu'**on procède à l'analyse d'une pluralité d'échantillons répartis sur la hauteur de la carotte pour caractériser les contaminants à différentes profondeurs.

3. Procédé d'analyse de la contamination de sols par des polluants, notamment organiques selon la revendication 1 **caractérisé en ce qu'**il comporte en outre des étapes d'analyse physique et/ou chimique d'une partie au moins desdits échantillons.

## Patentansprüche

1. Verfahren zum Analysieren der Verunreinigung von Böden durch Schadstoffe, insbesondere organische, zum Bestimmen des qualitativen und quantitativen Vorhandenseins von Bestandteilen von Interesse, die Schadstoffe bilden und insbesondere Kunststoffe, Kohlenwasserstoffe und Metalle umfassen, durch hyperspektrale Analyse der Reflexion und der Photolumineszenz, **dadurch gekennzeichnet, dass** die Analyse mit einer ersten Ausrüstung durch die Beleuchtung einer Probe durch eine Lichtquelle und durch mindestens einen Spektralsensor, der auf einem Spektrum von dem thermischen Infrarot bis zu dem Ultraviolett empfindlich ist, durchgeführt wird, **und dass** es aufweist:
- eine Sequenz zum Lernen, bestehend aus dem Vornehmen der Analyse einer Vielzahl von Referenzproben, und zum Speichern in einer Lerndatenbank
o a) der spektralen Reflexionssignatur, die durch Spektralanalyse erfasst wird
o b) bekannter Werte der Variablen, die die Verunreinigungen darstellen, die in jeder der Referenzproben vorhanden sind
o c) bekannter Werte der Variablen, die die Substrate jeder der Referenzproben darstellen
- eine Sequenz zum Kalibrieren einer Analyseausrüstung vor Ort relativ zu der ersten Ausrüstung, wobei die Ausrüstung vor Ort eine Lichtquelle und einen Spektralsensor aufweist,
- Sequenzen zum Analysieren einer Bodenprobe von einer geologischen Stelle, bestehend aus dem Erfassen der Reflexions- und/oder der Photolumineszenzsignatur der Probe unter Verwendung der so kalibrierten Ausrüstung vor Ort,
- und zum Vornehmen der Abschätzung der Charakterisierung der Schadstoffe durch Verarbeitung der Signatur durch eine Lernmaschine, die die Daten der Datenbank auswertet, die während der Lernsequenz gebildet werden.

2. Verfahren zum Analysieren der Verunreinigung von Böden durch Schadstoffe, insbesondere organische, nach Anspruch 1,
**dadurch gekennzeichnet, dass** man bei der Analyse einer Stelle mindestens eine Kernbohrung durchführt **und dass** man die Analyse einer Vielzahl von Proben, die über die Höhe des Bohrkerns verteilt sind, zum Charakterisieren der Verunreinigungen in verschiedenen Tiefen durchführt.

3. Verfahren zum Analysieren der Verunreinigung von Böden durch Schadstoffe, insbesondere organische, nach Anspruch 1,
**dadurch gekennzeichnet , dass** es ferner Schritte der physikalischen und/oder chemischen Analyse mindestens eines Teils der Proben aufweist.

## Claims

1. A method for analyzing, by means of hyperspectral analysis of a reflection and photoluminescence, soil contamination by pollutants, in particular organic pollutants, in order to determine the qualitative and quantitative presence of constituents of interest constituting pollutants and comprising in particular plastics materials, hydrocarbons and metals, **characterized in that** said analysis is carried out by means of a first item of equipment by illuminating a sample using a light source and by at least one spectral sensor sensitive to a spectrum ranging from thermal infrared to ultraviolet, and **in that** it includes:
- a sequence for learning, consisting in analyzing a plurality of reference samples, and for recording the following in a learning database
∘ a) the spectral signature of reflection acquired by spectral analysis
o b) known values of variables representative of the contaminants present in each of said reference samples
∘ c) known values of variables representative of substrates of each of said reference samples
- a sequence for calibrating a piece of field analysis equipment with respect to said first piece of equipment, said piece of field equipment including a light source and a spectral sensor,
- sequences for analyzing a soil sample from a geological site, consisting in acquiring the reflection and photoluminescence signature of said sample using said piece of field equipment thus calibrated,
- and in estimating the characterization of pollutants by processing said signature by means of a learning engine that uses the data from the database created during the learning sequence.

2. The method for analyzing soil contamination by pollutants, in particular organic pollutants, according to claim 1, **characterized in that,** during the analysis of a site, at least one core sampling operation is carried out, and **in that** the analysis of a plurality of samples distributed over the height of the core is carried out in order to characterize the contaminants at various depths.

3. The method for analyzing soil contamination by pollutants, in particular organic pollutants, according to claim 1, **characterized in that** it further includes steps of physical and/or chemical analysis of at least some of said samples.
